# EUROPÄISCHE PATENTANMELDUNG

(11) **EP 0 755 689 A1**
(43) Veröffentlichungstag der Anmeldung: **29.01.1997**
(21) Anmeldenummer: 96110151.6
(22) Anmeldetag: 24.06.1996
(51) Int. Cl.: A61M 5/30, A61M 37/00

(54) **Behandlung der erektilen Dysfunktion**

(30) Priorität: 25.07.1995 DE 19526984
(71) Anmelder: JENAPHARM GmbH, D-07745 Jena (DE)
(72) Erfinder: Dittgen, Michael, Prof. Dr., 95510 Apolda (DE); Hübler, Doris, Dr., 07407 Schmieden 12 (DE); Oettel, Michael, Prof. Dr., 07743 Jena (DE)
(74) Vertreter: Cramer, Eva-Maria

(57) **Zusammenfassung**

Die vorliegende Erfindung betrifft die Verwendung einer auf die Haut über den Schwellkörpern aufzusetzenden an sich bekannten Vorrichtung zur Einbringung pharmazeutischer Zubereitungen in den Schwellkörper zur Behandlung der erektilen Dysfunktion, der Impotenz und/oder verwandter Krankheitsbilder.
Die Vorrichtung besteht aus einem tubulären, einseitig offenen und in einem Mundstück zum Aufsetzen der Vorrichtung auf die Haut endenden Körper, wobei innerhalb des Körpers in der Reihenfolge vom geschlossenen Ende der Vorrichtung zum Mundstück hin eine Gaszylinder , ein Zylinderventil , ein Druckraum mit Membranen , eine Kapsel mit der pharmazeutischen Zubereitung und ein weiterer Druckraum zur Regulierung des Gasstromes und zur Geräuschminderung angebracht sind.
Durch manuellen Druck auf den Gaszylinder öffnet sich das Zylinderventil, die Membranen im Druckraum zerbersten, die pharmazeutische Zubereitung innerhalb der Kapsel wird beschleunigt und mit hoher Geschwindigkeit über den Druckraum zur Regulierung des Gasstromes und zur Geräuschminderung und das Mundstück durch die Haut der Schwellkörper geschossen.
Mit der Erfindung wird die Möglichkeit einer Behandlung der erektilen Dysfunktion, der Impotenz und/oder verwandter Krankheitsbilder aufgezeigt und entsprechend die Nachteile herkömmlicher Vorrichtungen vermieden.

## Beschreibung

Die Erfindung betrifft die Verwendung einer auf die Haut über den Schwellkörpern aufzusetzenden an sich bekannten Vorrichtung zur Einbringung pharmazeutischer Zubereitungen in den Schwellkörper zur Behandlung der erektilen Dysfunktion, der Impotenz und/oder verwandter Krankheitsbilder.

Die erektile Dysfunktion (Impotentia coeundi), das Unvermögen zur Erreichung oder zum Unterhalt einer penetrierungsfähigen Erektion des Penis in mindestens 75 % der Versuche, ist eine krankhafte Leistungsminderung, die hinsichtlich ihrer Inzidenz ab einem Alter von 50 Jahren dramatisch zunimmt. Mindestens ein Viertel der älteren Männer ist betroffen (in den USA mindestens 10 Millionen Männer).
Die häufigste Ursache sind arteriosklerotische Veränderungen der Penis-Arterie (Hofstetter A., Marchner M.: Erektile Dysfunktion - Aktuelle Diagnostik und Therapie. Bildgebung 58 (Suppl. 1): 35-37, 1991; Postgraduate Medicine 93: 65, 1994). Aus diesem Grund werden für eine Therapie vasoaktive Substanzen wie z.B. Apomorphin, Papaverin, Prostaglandin E₁ (Alprostadil), Phentolamin, Phenoxybenzamin, **V**asoaktives **I**ntestinales **P**olypeptid (VIP) und 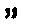 **C**alcitonin **G**ene **R**elated **P**eptide" (CGRP) allein oder in den verschiedensten Kombinationen untereinander angewendet. Die Applikationsroute ist mit sehr unsicherem Erfolg oral bzw. sublingual (Apomorphin-HCl) oder per (Selbst-) Injektion direkt in die Corpora cavernosa. Die derzeit häufigste Therapieform ist die **S**chwell**k**örper-**A**utoinjektions**t**herapie (SKAT) mit vasoaktiven Substanzen. In bestimmten Fällen werden auch Penis-Implantate (z.B. aufblasbar) empfohlen.
Die SKAT birgt eine Reihe von Nachteilen. Diese sind u. a.: Schmerzen bei der Injektion per Nadel, psychogene Hemmung bei Selbstinjektion (Nadelstich), Hämatombildung (incl. Ekchymose) an der Einstichstelle, Fibrosierung der Corpora cavernosa, Verwachsungen der Tunica albuginea, verlängerte Erektion/Priapismus (Th. l Sheng et al. Impotence evaluated by the use of prostaglandin E1. J Urol 141: 1357-1359, 1989; J Chen et al. Use of atomatic insulin injector for intracorporeal injection in erectile dysfunction. J Urol 152: 461-462, 1994; Ol Linet and LL Neff: Intracavernous prostaglandin E₁ in Erectile dysfunction. Clin Invest 72: 139-149, 1994; DKB Armstrong, AG Convery, WW Dinsmore: Reasons for patient drop-out an intracavernous autoinjection programme for erectile dysfunction. Br J Urol 74: 99-1O1, 1994). Außerdem wurde mehrfach darauf hingewiesen, daß auch mit der Zunahme der Dosis der lokale Schmerz bei der Injektion anwächst (Waldhauser M, Schramek P. Efficiency and side effects of prostaglandin E(1) in the treatment of erectile dysfunction. J Urol (Baltimore) 1988; 140: 525-7; Schramek P, Waldhauser M. Dosedependent effect and side-effect of prostaglandin E(1) in erectile dysfunction. Br J Clin Pharmacol 1989; 28: 567-71; Lee LM, et al. Prostaglandin E(1) versus phentolamine / papaverine for the treatment of erectile impotence: a double-blind comparison. J Urol (Baltimore) 1989; 141: 549-55.).

Somit lassen sich die Nachteile der bisherigen Behandlungsmöglichkeiten der erektilen Dysfunktion die auf einer Injektion beruhen, wie folgt zusammenfassen:
- die Verabfolgung ist schmerzhaft,
- es gibt eine Hemmschwelle für die Selbstapplikation,
- systemische Nebenwirkungen sind nicht auszuschließen,
- mit Hämatomen oder andere Nebenwirkungen an der Einstichstelle muß gerechnet werden.

Aus diesem Grund erscheint es nützlich und sinnvoll, nach Alternativen zur Injektion per Nadel (Stackl W, et al. Intracavernous injection of prostaglandin E(1) in impotent men. J Urol (Baltimore) 1988; 140: 66-8; Waldhauser M, Schramek P. Efficiency and side effects of prostaglandin E(1) in the treatment of erectile dysfunction. J Urol (Baltimore) 1988; 140: 525-7; Schramek P, Waldhauser M. Dosedependent effect and side-effect of prostaglandin E(1) in erectile dysfunction. Br J Clin Pharmacol 1989; 28: 567-71; Ishii N, et al. Intracavernous injection of prostaglandin E(1) for the treatment of erectile impotence. J Urol (Baltimore) 1989; 141: 323-5; Lee LM, et al. Prostaglandin E(1) versus phentolamine/papaverine for the treatment of erectile impotence: a double-blind comparison. J Urol (Baltimore) 1989; 141: 549-55.) bei der lokalen Applikation von Wirkstoffen in die Corpora cavernosa zu suchen.

Aus der Patentliteratur - Patentschrift WO 94/24236 A1 - ist eine Vorrichtung bekannt zur Verabreichung von Lokalanästhetika, beispielsweise Lidocain, im Bereich des Mundes und zur transdermalen Verabreichung von Insulin, Schmerzmittel und Chemotherapeutika.
Jedoch ist aus der Fach- und Patentliteratur die Anwendung dieser Vorrichtung zur Behandlung der erektilen Dysfunktion, der Impotenz und/oder verwandter Krankheitsbilder nicht bekannt und nicht schlußfolgernd.

Der Erfindung liegt die Aufgabe zugrunde, eine Vorrichtung zur Einbringung pharmazeutischer Zubereitungen zur Behandlung der erektilen Dysfunktion, der Impotenz und/oder verwandter Krankheitsbilder aufzuzeigen und die Nachteile herkömmlicher Vorrichtungen zu vermeiden.

Die Aufgabe wird erfindungsgemäß durch die Verwendung einer auf die Haut über den Schwellkörpern aufzusetzenden an sich bekannten Vorrichtung zur Einbringung pharmazeutischer Zubereitungen in den Schwellkörper zur Behandlung der erektilen Dysfunktion, der Impotenz und/oder verwandter Krankheitsbilder gelöst.

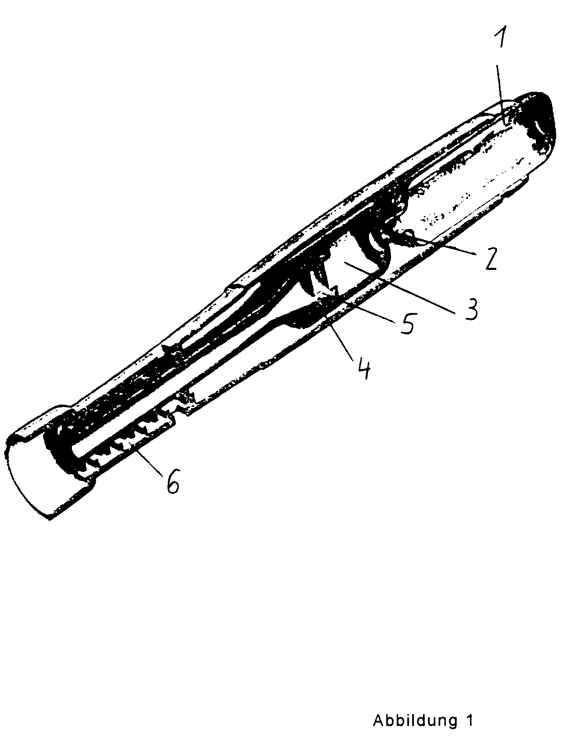

Die Vorrichtung nach Abbildung 1 besteht aus einem tubulären, einseitig offenen und in einem Mundstück zum Aufsetzen der Vorrichtung auf die Haut endenden Körper, wobei innerhalb des Körpers in der Reihenfolge vom geschlossenen Ende der Vorrichtung zum Mundstück hin eine Gaszylinder 1, ein Zylinderventil 2, ein Druckraum mit Membranen 3, eine Kapsel 4 mit der pharmazeutischen Zubereitung 5 und ein weiterer Druckraum zur Regulierung des Gasstromes und zur Geräuschminderung 6 angebracht sind.

Durch manuellen Druck auf den Gaszylinder 1 öffnet sich das Zylinderventil 2, die Membranen im Druckraum 3 zerbersten, die pharmazeutische Zubereitung 5 innerhalb der Kapsel 4 wird beschleunigt und mit hoher Geschwindigkeit über den Druckraum zur Regulierung des Gasstromes und zur Geräuschminderung 6 und das Mundstück durch die Haut der Schwellkörper geschossen.
Die Beschleunigung der pharmazeutischen Zubereitung erfolgt dabei mittels unter Druck stehenden Gases.
Sie ist aber auch mittels gespannter Federn, durch Erhitzen, durch Quellung, piezoelektrisch oder motorisch zu erreichen.

Es wurde überraschenderweise gefunden, daß mindestens ein potenzsteigernder Wirkstoff, wie Prostaglandin E1 (Alprostadil), Papaverin, Phentolamin, Calcitonin Gene Related Peptide (CGRP), Vasoaktives intestinales Polypeptid (VIP) praktisch schmerzfrei und weitgehend vollständig in die Schwellkörper gelangen, wenn sie als geeignete Zubereitung mittels Druck durch die Haut hindurch appliziert werden.

Besonders sind feste Lösungen geeignet, die mindestens zu 10% den Wirkstoff molekulardispers enthalten. Dabei ist es gleichgültig, welcher Feststoff zur Herstellung der festen Lösungen verwendet wird. Allerdings muß der Festoff unter dem Aspekt der Schmerzfreiheit ausgewählt werden und weitere Hilfsstoffe, wie Stabilisatoren, Puffersubstanzen usw. sollten nur zu einem geringen Anteil enthalten sein.
Als geeignete Feststoffe haben sich bestimmte Salze und/oder Zucker, Alkohole und/oder Tenside in geringer Konzentration erwiesen.
Es können aber auch Feststoffe, wie Harnstoff, Nicotinamid, Bernsteinsäure, Poly-(lactid)-, Poly-(lactid-co-glycolid)-Verbindungen usw. eingesetzt werden. In diesen Fällen hat es sich als besonders vorteilhaft erwiesen, wenn der Wirkstoff zusammen mit dem geeigneten Feststoff nach einem speziellen Verfahren (Verfahren und Vorrichtung zur Herstellung fester Dispersionen, Anmeldung P 44 06 462.4-41) zur festen Dispersion verarbeitet wird.

Die nachfolgenden Beispiele sollen die Erfindung in nicht beschränkender Weise erläutern:

### Beispiel 1

### Feste Lösung mit Papaverin und Phentolamin

| | |
|---|---|
| Papaverin | 1,00 g |
| Phentolamin | 0,02 g |
| stabilisierender Zusatz | 0,01 g |
| Poly-(lactid-co-glycolid) ad | 2,00 g |

Die Mischung wird in 2-ml-Vials abgefüllt und sterilisiert. 100 µl dieser Mischung werden mit einer geeigneten Vorrichtung aufgenommen und anschließend mittels Druck durch die Haut hindurch direkt in die Schwellkörper appliziert

### Beispiel 2

### Feste Lösung mit Alprostadil

| | |
|---|---|
| Alprostadil | 0,400 mg |
| Harstoff | 0,950 mg |
| Fructose | 0,750 mg |
| Ethanol* | 0,050 mg |

| | |
|---|---|
| * in der fertigen Zubereitung nicht mehr enthalten | |

Eine Mischung der Substanzen wird, wie in Anmeldung P 44 06 462.4-41 angegeben, mit 500 U/min und kurzzeitiger Anwendung von 180 °C in eine feste Lösung überführt. Die feste Lösung wird in 5-ml-Vials abgefüllt und sterilisiert.
100 µg dieser festen Lösung werden mit einer geeigneten Vorrichtung aufgenommen und anschließend mittels Druck durch die Haut hindurch direkt in die Schwellkörper appliziert.

Gegebenfalls besteht auch die Möglichkeit, die feste Substanz zunächst mit 2 ml physiologischer Kochsalzlösung in Lösung zu bringen und von dieser Lösung dann 100 µl wie bereits beschrieben mittels Druck zu applizieren.

### Beispiel 3

### Feste Lösung mit Alprostadil und CGRP

| | |
|---|---|
| Alprostadil | 0,40 mg |
| CGRP | 0,20 mg |
| stabilisierender Zusatz | 0,01 mg |
| Poly-(lactid-co-glycolid) ad | 2,00 g |

Die Mischung wird in 2-ml-Vials abgefüllt und sterilisiert. 100 µl dieser Mischung (entsprechend 20 µg Alprostadil und 10 µg CGRP) werden mit einer geeigneten Vorrichtung aufgenommen und anschließend mittels Druck durch die Haut hindurch direkt in die Schwellkörper appliziert.

### Beispiel 4

### Feste Lösung mit Alprostadil

| | |
|---|---|
| Alprostadil | 0,40 mg |
| stabilisierender Zusatz | 0,01 mg |
| Harnstoff/Zuckeralkohol ad | 2,00 g |

Die Mischung wird in 2-ml-Vials abgefüllt und sterilisiert. 100 µl dieser Mischung (entsprechend 20 µg Alprostadil) werden mit einer geeigneten Vorrichtung aufgenommen und anschließend mittels Druck durch die Haut hindurch direkt in die Schwellkörper appliziert.

Nachfolgend wird als Beispiel das Ergebnis einer klinischen Studie dargestellt.

Als Zubereitungen werden feste Lösungen nach Beispiel 3 und 4 untersucht. Die pharmazeutischen Zubereitungen werden in die an sich bekannte Vorrichtung zur nadelfreien Applikation eingebracht. Diese Vorrichtung wird fest auf die dorso-laterale Penishaut (etwa in Mitte der Gesamt-Penislänge)gedrückt, wobei auf eine senkrechte Haltung gegenüber dem Penisschacht geachtet wird. Es wird die pharmazeutische Zubereitung beschleunigtpplikation fest auf die dorso-laterale Penishaut (etwa in Mitte der Gesamt-Penislänge) gedrückt. Dabei wird auf eine senkrechte Haltung gegenüber dem Penisschaft geachtet. Es wird die pharmazeutische Zubereitung beschleunigt und mit hoher Geschwindigkeit durch die Haut in den Schwellkörper geschossen. Die Beschleunigung erfolgt hierbei durch selbstregulierenden Luftdruck.
In Versuchsreihen wird der Luftdruck ermittelt, bei dem die Eindringtiefe der pharmazeutischen Zubereitung bei geringem Schmerzempfinden optimal ist. Durch Variation von Ort und Gestalt der Düse der nadelfreien Vorrichting konnte ebenfalls Einfluß auf eine schmerzarme Applikation der pharmazeutischen Zubereitung genommen werden.

Die Studie wurde mit 14 Männern mit organisch bedingter erektiler Dysfunktion im Alter von 52 bis 63 Jahren durchgeführt. Mindestens einen Monat vor Studienbeginn war mittels SKAT bzw. Alprostadil-Nadelinjektion eine Ansprechbarkeit bei allen Patienten festgestellt worden. Es wurden drei verschiedene Zubereitungen getestet:
- Medikation A:: Physiologische Kochsalzlösung
- Medikation B:: Zubereitung nach Beispiel 4, 20 µg Alprostadil pro dosi
- Medikation C:: Zubereitung nach Beispiel 3, 10 µg CGRP plus 20 µg Alprostadil pro dosi.

Das Injektionsvolumen von 100 µg war bei allen drei Medikationen gleich. Jeder Patient applizierte sich unter Aufsicht eines vom Studienleiter bestimmten klinischen Monitors an jedem Test-Tag je eine Dosis in den rechten und linken Penisteil. An jedem Testtag benutzte jeder Patient nur eine Medikation. Am zweiten Testtag wurde die nächste Medikation und am dritten Testtag wurde die dritte, d.h. letzte Medikation selbst appliziert. Die einzelnen Testtage lagen genau eine Woche auseinander. Die Studie wurde mit verblindeter Medikation und randomisiert durchgeführt, d.h. weder Arzt, noch Monitor, noch Patient kannten die Zusammensetzung der jeweilig eingesetzten Medikation.

2 Patienten verließen nach den ersten beiden Injektionen ohne Angabe von Gründen die Studie, so daß insgesamt 12 Patienten ausgewertet werden konnten.
Nach jeder Injektion wurden die Patienten nach ihrem subjektiven Befinden befragt, vor allem nach Schmerzgefühlen. Es wurden 3 Variable erfasst.

### Variable 1:

Dauer der Erektion in min. Es wurde die Zeitdauer zwischen dem Eintreten einer mehr als 8O %igen Erektion (verglichen mit dem Maximum) und dem Nachlassen der Penis-Erektionshöhe unter 8O % des Maximums für mindestens 2O Minuten bestimmt.

### Variable 2:

Subjektive Einschätzung der Rigidität des Penis durch Selbsteinschätzung ( Prozentsatz im Vergleich zu den vom Patienten bisher selbst beobachteten Höchstwert).

### Variable 3:

Bestimmung der objektiven Penis-Rigidität mittels Rigi-Scan (Dacomed Corporation, Minneapolis/USA). Es wurden zwei Schleifen-Transducer benutzt. Einer an der Peniswurzel und einer in der Höhe des Sulcus. Die Rigiscan-Rigiditätsmessung erfolgte in Prozentangaben, wobei 1OO % einer Rigidität eines Holzdübels entsprach. Eine Rigidität von 6O % ist für die Penetration der Vagina ausreichend. Die Rigidität wurde aller 5 Minuten, beginnend 15 Minuten vor der nadelfreien Injektion und endend 1 Stunde danach gemessen.

Die Ergebnisse sind in nachfolgenden Tabelle dargestellt.

### Ergebnisse der vergleichenden Studie Medikation A vs. B vs. C

| Behandlung | n | Mittlere Erektionsdauer (min) +/- S.E.M. | Rigidität (%) - subjektiv -+/- S.E.M. | Rigidität (%) -Rigiscan-+/- S.E.M. |
|---|---|---|---|---|
| Physiol. NaCl (Kontrolle) | 12 | keine Reaktion | 2O.2 +/-5.8 | 17.3 +/- 4.8 |
| 2O µg PGE1 | 12 | 12.3 +/- 4.2 * | 62.4 +/- 11.3 * | 61.O +/-1O.5* |
| 2O µg PGE1 plus 1O µg CGRP | 12 | 25.7 +/-9.9** | 74.6 +/-15.2** | 77.O +/-12.8** |

| | | | | |
|---|---|---|---|---|
| * < 0.05 vs. NaCl | | | | |
| ** < 0.05 vs. PGE1 alone and < O.OO1 vs. NaCl ( Fisher-Test) | | | | |

Die vergleichende Studie zeigte einerseits klar, daß sich beide untersuchten Wirkstoffe hinsichtlich Erektionsdauer und Rigidität signifikant von der Kontrolle absetzen. Die zusätzliche und simultane Applikation von Calcitonin Gene Related Peptide (CGRP, Medikation C) verbessert die Ergebnisse im Vergleich zur PGE1- Applikation allein. Die Unterschiede sind ebenfalls signifikant.

Zu betonen ist weiterhin, daß weder systemische noch lokale Nebenwirkungen beobachtet bzw. mitgeteilt wurden. Die Patienten bestätigten übereinstimmend die im Vergleich zur Nadel-Injektion angenehme und schmerzarme Anwendung.

### Bezugszeichen

- 1: Gaszylinder
- 2: Zylinderventil
- 3: Druckraum mit Membranen
- 4: Kapsel
- 5: pharmazeutischen Zubereitung
- 6: Druckraum zur Regulierung des Gasstromes und zur Geräuschminderung

## Patentansprüche

1. Verwendung einer auf die Haut über den Schwellkörpern aufzusetzenden an sich bekannten Vorrichtung zur Einbringung pharmazeutischer Zubereitungen in den Schwellkörper
zur Behandlung der erektilen Dysfunktion, der Impotenz und/oder verwandter Krankheitsbilder.
